# EUROPEAN PATENT SPECIFICATION

(11) **EP 2 111 198 B1**
(45) Date of publication and mention of the grant of the patent: **18.06.2014**
(21) Application number: 08700549.2
(22) Date of filing: 07.02.2008
(51) Int. Cl.: A61F 9/008

(54) **OPHTHALMOLOGICAL APPARATUS FOR BREAKDOWN OF EYE TISSUE**
OPHTHALMOLOGISCHES GERÄT ZUR DISRUPTION VON AUGENGEWEBE
APPAREIL OPHTALMOLOGIQUE POUR ROMPRE DU TISSU OCULAIRE

(30) Priority: 14.02.2007 WO PCT/CH2007/000078; 14.02.2007 US 901083 P
(43) Date of publication of application: 28.10.2009
(60) Divisional application: 12006586.7
(73) Proprietor: Ziemer Holding AG, 2562 Port (CH)
(72) Inventor: RATHJEN, Christian, 28197 Bremen (DE)
(74) Representative: Rentsch Partner AG
(86) International application number: PCT/CH2008/000045
(87) International publication number: WO 2008/098388

(56) References cited:
- EP-A- 1 486 185
- EP-A- 1 731 120
- DE-A1-102005 001 249
- US-A- 5 364 390
- US-A1- 2003 208 189

## Description

### Technical field

The present invention relates to an ophthalmological apparatus for breakdown of eye tissue. The invention relates in particular to an ophthalmological apparatus that comprises a base station with a light source for generating light pulses, an application head that can be mounted on the base station by means of a support arm and can be placed onto the eye, with a light projector for focused projection of the light pulses, and an optical transmission system for transmitting the light pulses from the base station through the support arm to the application head.

### Prior art

Instances of ametropia such as myopia (short-sightedness), hyperopia (long-sightedness or far-sightedness) or astigmatism can nowadays be permanently corrected by refractive surgical treatment. Refractive surgical treatments are surgical operations on the eye which change the optical refractive power of the eye with the aim of bringing it as close to a desired value as possible. One of the most important methods in refractive surgery is so-called laser-assisted in situ keratomileusis (LASIK) in which the interior of the cornea is removed with the aid of a computer-controlled excimer laser after a corneal flap has previously been partially severed and folded aside. To produce the corneal flap, use is made of mechanical microkeratomes in which a driven scalpel cuts the corneal flap. Recently, such corneal flaps have also been cut with the aid of strongly focused femtosecond laser pulses, which have pulse widths of typically 100 fs to 1000 fs (1 fs = 20⁻¹⁵s). In addition to LASIK, there are further procedures for refractive correction that are performed on the cornea with the aid of femtosecond lasers.

Such a system is marketed, for example, by IntraLase Corp, in Irvine, California, USA under the name of Pulsion FS Laser. In this system, a light source (femto laser) is located in a base station and is connected to an optical application head via an articulated mirror arm. In addition to the optical transmission system, a deflection system (scanner) and the light projector, the application head also comprises viewing means, such as camera or surgical microscope, and therefore has a considerable weight of several kilograms. For orienting and applying the light projector onto an eye of a patient, the entire application head is moved via linear translatory drives. The patient lies on a bed and does not move. For reasons of weight, the application cannot be done by hand, and motorized drives are necessary. To avoid applying excessive forces to the patient, the laser system comprises force-measuring systems in the vertical application direction.

Systems for cutting the corneal flap with focused femtosecond laser pulses are also marketed by Zeiss Meditec AG, with its Visumax, and by 20/10 Perfect Vision Optische Geräte GmbH, with its Femtec. In these systems, the light projector is connected fixedly to the base station. In these systems, the patient is oriented in the horizontal plane and also vertically with respect to the light projector with the aid of a patient bed. Therefore, these systems too cannot be applied manually onto the patient's eye. In addition, the special patient bed has to be integrated into the safety system of the laser system in order to avoid uncontrolled movements of the bed. The bed is thus part of the laser system and thus increases the system costs and the space required. A suitable choice of bed by the person using the system is not possible.

In the aforementioned systems, the docking of the light projector onto the patient's eye is first effected by orientation in the horizontal plane in the x-direction and y-direction (centring of the eye) and then by lowering or lifting the patient in the vertical z-direction.

EP 1731120 describes a system in which the application head is mounted flexibly on the base station via an articulated mirror arm composed of several arm elements and joints and permits manual application of the application head and light projector onto the eye of a patient. To permit the weight of the application head for manual application by means of the articulated mirror arm, the light projector has smaller lens systems compared to the previously known systems. In order to ensure that, despite the lens system of smaller dimensions, it is possible to work with focused laser pulses across an extensive work area on the eye, the application head additionally has movement drivers for moving the light projector in an advance direction and in a first scanning direction. An optical deflection to a vertical scanning direction is not possible, however. The limited work area permits only rapid positioning of the laser pulses within the work area. Limits are thus set on flexible positioning of the laser pulses across the entire eye.

DE 102005001249 discloses a security mechanism which allows movement of the contact lens in direction away from the eye to prevent a dangerous increase in intraocular pressure due to eye movement during the surgical procedure.

### Disclosure of the invention

It is an object of the present invention to propose a novel ophthalmological apparatus for breakdown of eye tissue, which apparatus avoids at least some disadvantages of the prior art. A particular object of the present invention is to make available an ophthalmological apparatus for breakdown of eye tissue which enables manual application of the light projector onto an eye of a patient but permits a light projector with optics of high numerical aperture, which permits the focused projection of laser pulses in the whole viewable eye area both in the horizontal and also the vertical direction.

According to present invention, these objects are achieved in particular by the elements of independent claim 1. Further advantageous embodiments are also set forth in the dependent claims and the description.

The ophthalmological apparatus comprises a base station with a light source for generating light pulses, a support arm mounted on the base station, an application head that is mounted on the support arm and can be placed onto an eye and has a light projector for focused projection of the light pulses for punctiform breakdown of eye tissue, an optical transmission system for transmitting the light pulses from the base station through the support arm to the application head and beam-deflecting means, arranged in the application head and configured to deflect the light pulses in at least two scanning directions.

The aforementioned objects are achieved in the present invention particularly by virtue of the fact that the support arm is of rigid design with a horizontal orientation and, at one end, has a hinge (in German "Drehgelenk") with an essentially horizontally oriented rotation axis, the hinge being mounted in such a way that the application head can be placed onto the eye with a rotation (rotation movement) extending about the rotation axis for lowering and placing the application head onto the eye. The rigid support arm, i.e. designed in one piece and inherently immovable, permits a stable and simple connection of the application head to the base station. In particular, the rigid support arm and its constant, defined horizontal orientation make it possible to design the optical transmission system more simply than is the case, for example, with a multi-part articulated mirror arm. The hinge arranged horizontally on an end of the rigid support arm permits application of the application head or of the light projector onto the eye in the vertical direction via a preferably mechanically executed rotation movement, wherein the application head is guided on a kinematically generated arc, which permits a vertical movement component for placement onto the eye. The advantage of the rotation movement is its simple configuration and minimal mechanical friction. The support arm and the hinge thus permit controlled manual docking, i.e. application of the application head or light projector onto the eye, the user advantageously being able to employ his tactile senses in order to control the force or movement exerted via the tactile force and movement feedback. Because active systems for applying force and limiting force are unnecessary, increased safety is achieved only through the use of passive system elements. On account of the simply designed support arm and the actuators and sensors required only in small numbers, or not at all, a further simplified configuration and therefore lower costs are achieved. Moreover, the overall size of the components located directly over the patient can be reduced.

The hinge is preferably arranged on that end of the support arm directed away from where the support arm is mounted on the base station, and the application head is connected movably to the support arm via the hinge. By means of the movable arrangement of the application head on the rigid support arm via a hinge, the size and weight of the system part to be applied manually is reduced, which further facilitates the manual application and makes it controllable.

Preferably, the hinge is mounted in such a way that the application head can be rotated into a vertical position. Placing the application head in a vertical, upright position has the advantage that the light projector is easier accessible to an operator for maintenance or preparation, e.g. for cleaning, mounting alternative projection and/or contact elements, and/or attaching disposable protection covers.

In an embodiment, the apparatus comprises positioning means for rotary and/or translatory movement of the support arm parallel to a positioning plane for the positioning of the application head over the eye. The positioning means comprise, for example, guide rails and/or drive means for translatory movements in a horizontal positioning plane and/or hinges that permit a rotation (rotation movement) about a vertically extending rotation axis.

In an embodiment the horizontally oriented hinge is connected to the support arm via a further hinge. The further hinge has a vertically oriented rotation axis and enables a rotation of the application head about this vertical rotation axis. Using the vertically oriented hinge for connecting the horizontally oriented hinge to the support arm makes it possible to position and place the application head or the light projector, respectively, through simple and defined mechanical rotation movements about the vertical and horizontal axis. Furthermore, when placed in a vertical, upright position, the application head can additionally be rotated away from the patient towards the operator for easier maintenance and preparation operations.

In another embodiment, the support arm is connected movably to the base station via the horizontal oriented hinge, the hinge being implemented as a pivot hinge or a pivot joint, for example. The horizontally oriented rotation axis is aligned with the longitudinal axis of the support arm and enables a rotation of the support arm about its longitudinal axis. Thus, by rotating the support arm about its longitudinal axis, the application head or the light projector, respectively, can be placed onto the eye through a simple and defined mechanical rotation movement.

In an embodiment, the application head is connected to the support arm via a further hinge. The further hinge has a rotation axis oriented perpendicularly to the longitudinal axis of the support arm and enables a rotation of the application head about this vertical rotation axis. The hinge oriented perpendicularly to the support arm makes it possible to position the application head over the eye through rotary movement of the support arm.

In an embodiment the support arm is connected to the base station via a pivot joint having a vertically oriented rotation axis and enabling a rotation of the support arm about this vertical rotation axis. Connecting the support arm with the pivot joint to the base station provides a further degree of flexibility for positioning and placing the support arm and, thus, the application head and the light projector.

In an embodiment, the apparatus comprises beam-deflecting means arranged in the support arm and used for deflecting the light pulses in at least two scanning directions, and the light projector is dimensioned such that, without mechanical movement of the light projector, deflected light pulses can be projected in a focused manner across an entire working area of preferably 11 mm in diameter for breakdown of eye tissue.

In another embodiment, the application head has a viewing window permitting a top view of the eye in the projection direction of the light projector. The viewing window permits a top view of the eye by way of optical viewing or measuring modules, e.g. cameras, microscopes, measuring devices, which are connected movably to the base station or, alternatively, are independent from the base station. The optical viewing or measuring modules are oriented in the projection direction of the light projector and can be swivelled over the viewing window, or vice versa, while the application head is placed on or is being placed onto the eye. Moreover, the optical viewing or measuring modules can be swivelled away when not required. The possibility of optical viewing or measuring modules being able to be swivelled into and out of the line of sight above the eye means that the overall size and therefore the weight of the application head can be further reduced and its handling improved.

In an embodiment, weight compensation means are connected to the application head, in order to partially balance out the application head above the rotation axis in such a way that the application head can be placed onto the eye with an application force reduced by the inherent weight of the application head. By means of the only partially balanced out configuration of the masses involved in the rotation movement (application head and, depending on the design, the support arm), the application force on the eye can be generated by gravity. In an embodiment, the compensation means are adjustable by the operator, e.g. for full or exceeding compensation of the application head so that any force applied to the eye is provided manually by the operator.

In another embodiment, the light projector comprises a first lens system in a projection part of the application head oriented in the projection direction, and the light projector comprises a second lens system in a feed part of the application head angled away from the projection part and directed towards the support arm, said first and second lens systems being coupled via a deflecting mirror. The arrangement of the lens systems divided up into the projection part and feed part permits a reduction of the structural height of the light projector and thus of the application head. Moreover, external aids such as microscopes, cameras, visual aids onto which the patient can fixate his eyes and/or aiming aids, or measuring devices can be adapted more easily.

In an embodiment, the application head comprises a projection part oriented in the projection direction and a feed part angled away from the projection part and directed towards the support arm. The projection part is connected rotatably to the feed part, e.g. axially about a longitudinal axis extending through the feed part and/or about a transverse axis extending perpendicular to the longitudinal axis of the feed part. The rotatable connection of the projection part to the feed part allows light projector and securing means mounted thereon to be oriented precisely with the eye.

In another embodiment, the apparatus comprises a lock for fixing or releasing a rotation position of the application head about the rotation axis.

In one embodiment, the apparatus comprises height-defining means for determining a vertical position of the eye, and the base station comprises height-positioning means for setting a vertical basic position of the support arm. By setting a vertical basic position of the support arm, the rotation movement needed for vertical placement of the application head onto the eye can be reduced and thus be kept in an area closer to the vertical movement component, which on the one hand reduces the movement stroke necessary for the docking of the application head and on the other hand facilitates the horizontal orientation of application head and eye (centring).

In other embodiments, the application head has one or more grips and grip structures for manual handling, a contact body which can be placed onto the eye, is transparent at least in parts and is configured and arranged such that it sets a contacted area of the eye equidistant to a working surface, and securing means for fixing the application head on the eye by underpressure. The light source preferably comprises a femtosecond laser.

In a further embodiment, the apparatus comprises a column mounted on the base station. The support arm is mounted on the column, and the column is vertically movable for setting a vertical position of the support arm.

In an embodiment, the application head is formed as an elongated member. Integrated in the elongated member are counter-weights for balancing out at least partially the application head above the horizontally oriented rotation axis. For example, beam-deflecting means arranged in the elongated member can be used advantageously as counter-weights for balancing the application head. The elongated member is connected to the support arm via a hinge having a rotation axis oriented perpendicularly to the longitudinal axis of the support arm. The latter hinge enables lateral movement of the elongated member, and thus the application head, parallel to the longitudinal axis of the support arm.

### Brief description of the drawings

An embodiment of the present invention is described below by way of example. The illustrative embodiment is depicted in the attached figures, in which:
Figure 1 shows a schematic side view of the ophthalmological apparatus, comprising a one-piece design of a base station with a support arm on which an application head is mounted rotatably.
Figure 2 shows a schematic side view of an ophthalmological apparatus comprising a support arm which can be moved in translation relative to the base station in a horizontal positioning plane and on which the application head is mounted rotatably.
Figure 3 shows a schematic top view of an ophthalmological apparatus comprising a support arm which can be moved in translation relative to the base station in the positioning plane and on which the application head is mounted rotatably.
Figure 4 shows a schematic top view of an ophthalmological apparatus comprising a support arm which can be moved in translation and rotation relative to the base station in the positioning plane and on which the application head is mounted rotatably.
Figure 5 shows a schematic top view of an ophthalmological apparatus comprising a support arm which can be moved in rotation relative to the base station about a vertical axis of rotation in the positioning plane and on which the application head is mounted rotatably.
Figure 6 shows a schematic top view of another ophthalmological apparatus comprising a support arm which can be moved in rotation relative to the base station in the positioning plane about a vertical axis of rotation and on which the application head is mounted rotatably.
Figure 7 shows a schematic side view of the application head, which comprises one lens system in a feed part to the support arm and another lens system in an angled projection part.
Figure 8 shows a schematic side view of the application head, comprising a viewing window over the projection part, to which an optical viewing aid or measuring module is coupled in a releasable manner.
Figure 9 shows a schematic side view of the application head, comprising a projection part connected rotatably to the feed part.
Figure 10 shows a schematic top view of another ophthalmological apparatus comprising a support arm which can be moved into a vertical, upright position through rotation.
Figure 11 shows a three-dimensional view of another ophthalmological apparatus positioned next to a schematically illustrated bed with a patient.
Figure 12 shows a three-dimensional view of the ophthalmological apparatus of Figure 11, with a support arm that can be rotated about its longitudinal axis.
Figure 13 shows a three-dimensional view of the ophthalmological apparatus illustrated in Figure 10.
Figure 14 shows a three-dimensional view of the ophthalmological apparatus illustrated in Figure 14 from a different viewing angle.

### Ways of implementing the invention

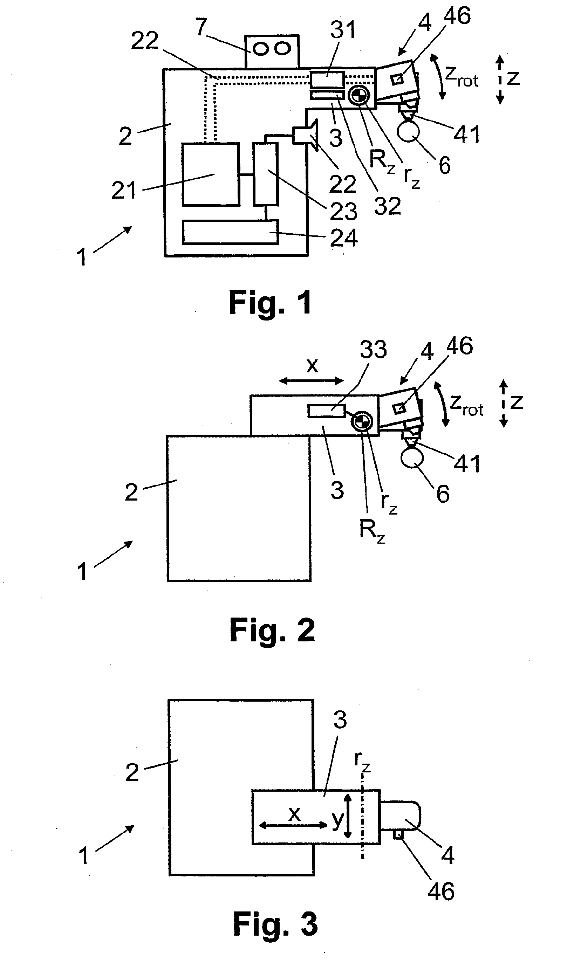

In Figures 1 to 6, as well as Figures 10 to 14, reference number 1 designates an ophthalmological apparatus for breakdown of eye tissue. The ophthalmological apparatus 1 comprises a base station 2 and an inherently rigid horizontal support arm 3 mounted thereon. For transporting the ophthalmological apparatus 1, the base station 2 is provided with lockable rolls or wheels. An application head 4 is mounted on the support arm 3 fixedly or via a horizontally oriented hinge R_{z}. Figure 1 also shows an optional optical viewing, imaging and/or measuring module 7 connected fixedly to the support arm 3, for example a monitor and/or a microscope for observing the application procedure (docking) and the treatment. In the embodiments according to Figures 1 to 6, 10, 13, and 14, the application head 4 is connected to the support arm 3 via the horizontally oriented hinge R_{z} so as to rotate about the horizontal rotation axis r_{z}. The horizontally oriented hinge R_{z} is in each case of these embodiments arranged at that end of the support arm 3 directed away from the connection of the support arm 3 to the base station 2. As is shown schematically in the side views in Figures 1 and 2, the hinge R_{z} is arranged and configured such that, by the rotation zᵣₒₜ of the application head 4 about the rotation axis r_{z}, a vertical movement component can be executed in the z-direction for lowering and placing the application head 4 onto an eye 6. For manual handling, grips and/or grip structures 46 are mounted on the application head 4. In an embodiment, in order to keep the mass inertia low, and to reduce the number of the moved components, the rotation axis r_{z} is arranged close to the patient. A person skilled in the art will understand that the rotation of the application head 4 about the rotation axis r_{z} can also be effected by means of a parallelogram guide, for example, with further hinges R_{z} being used for this purpose.

Although this is only shown schematically in Figure 1, the ophthalmological apparatus 1 in each case comprises a light source 21 (laser source) which is arranged in the base station 2 and generates light pulses, in particular a femtosecond laser for generating femtosecond laser pulses, and an optical transmission system 22 for transmitting the light pulses from the base station 2 through the support arm 3 to the application head 4. The ophthalmological apparatus 1 preferably also comprises a lock 32 for fixing or releasing a position of rotation of the application head 4 about the rotation axis r_{z}, for example a friction coupling a catch or another engaging element that can be activated and released selectively.

Beam-deflecting means 31 are inserted into the optical transmission system 22 and are designed to deflect the light pulses in at least two scanning directions. A scanner suitable for the beam-deflecting means 31 is described in WO 2007/056486, for example. Galvanoscanners or acousto-optic modulators are also suitable. The beam-deflecting means 31 are preferably arranged in the support arm 3 and/or in the application head 4 in order to minimize the distance to the treatment area. For example, optical components for adjusting the (vertical) projection focus, e.g. a movable lens, are mounted in the support arm 3, while a galvanoscanner, for deflecting the laser beam in two (horizontal) scanning directions, is mounted in the application head 4. In an alternative example, the beam-deflecting means 31 include an additional high scanning device, mounted in the support arm 3, which allows for the superposition of a directionally adjustable microscan to the normal scan pattern, as described in EP 1 731 120, for example. The application head 4 comprises a light projector 41 with a lens system 42, 43 for focused projection of the light pulses into and/or onto the eye 6, in order to effect a punctiform breakdown of the eye tissue. The deflected light pulses are transmitted onwards by the optical transmission system 22 via the beam-deflecting means 31 to the light projector 41. The beam-deflecting means 31 and the light projector 41 are designed to scan a contiguous work area extending across the entire viewable region of the eye and to work with a focusing effect such that contiguous sections in the eye tissue, in particular in the cornea, can be cut. The beam-deflecting means 31 and the light projector 41 not only permit focused scanning of a plane work region, for example a horizontal work region, but, by targeted vertical positioning of the focus, also three-dimensionally defined work regions, for example vertical and curved sectional surfaces.

Although this is not shown in the figures, it should be noted that the application head 4 comprises a contact body which can be placed onto the eye 6, is transparent to light at least in parts and is configured and arranged such that it sets a contacted area of the eye 6 preferably equidistant to a work surface, and has securing means for fixing the application head 4 to the eye 6 by underpressure. Contact bodies can be plane or spherical, for example.

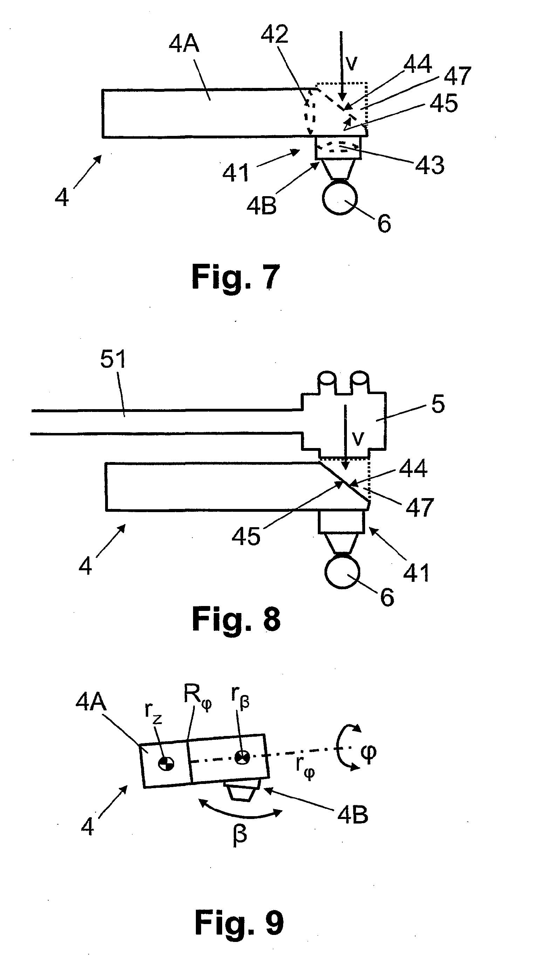

As is shown schematically in Figure 7, the application head 4, in an embodiment that can be combined with Figures 1-6 and Figures 10-14, has a feed part 4A connected to the support arm 3, and a projection part 4B angled off from the feed part 4A. In this embodiment, the light projector 41 comprises a first lens system 43 in the projection part 4B and a second lens system 42 in the feed part 4A. The first lens system 43 and the second lens system 42 are coupled via the deflecting mirror 45. The application head 4 preferably also has a viewing window 44 permitting a top view of the eye 6 in the projection direction v. The viewing window 44 is designed, for example, such that the deflecting mirror 45 is transparent to light for the viewing wavelength. Reference number 47 designates coupling means, which are described in more detail below.

As is shown schematically in Figure 8, the viewing window 44 permits optical coupling of optical viewing, imaging and measuring modules 5, e.g. a recording camera with optional pivotable monitor, which are mounted, for example by way of a module support 51, on the base station 2 so as to rotate about a vertically oriented rotation axis. These modules can thus be swivelled in and out over the application head 4, and their optical axes can be oriented with respect to the optical projection axis v of the light projector 41. The modules can additionally be mechanically connected to the application head 4 in a releasable manner via the coupling means 47, for example a detachable snap-fit catch or a bayonet catch. The coupling means 47 make it possible to connect mechanically and optically to the ophthalmological apparatus 1 the optical viewing, imaging and measuring modules 5 placed over the viewing window 44. The additional weight of these add-on modules can be compensated by setting correspondingly the adjustable weight compensation means 33, e.g. the adjustable counter-weights 33'.

In an optional embodiment that can be combined with Figures 1-8, and 10-14 and is shown schematically in Figure 9, the projection part 4B is mounted pivotably on the feed part 4A. In the variant according to Figure 9, the application head 4 has a hinge Rϕ and the projection part 4B is rotatably connected with the angle ϕ about a longitudinal axis r_{ϕ} extending through the feed part 4A. The application head 4 additionally has a hinge R_{β} and the projection part 4B is rotatably connected to the feed part 4A with the angle β about a transverse axis r_{β} extending perpendicular to the longitudinal axis r_{ϕ}. Preferably, e.g. for mounting different projection objectives and/or contact and/or fastening elements, the ophthalmological apparatus 1 comprises a lock for fixing or releasing the position of rotation of the projection part 4B about the rotation axis r_{ϕ}, for example a friction coupling, a catch or another engaging element that can be activated and released selectively.

In the embodiment according to Figure 1, the support arm 3 is fixedly connected to the base station 2, for example the base station 2 and the support arm 3 are designed in one piece as an overall unit with a common housing. In this variant, the mutual orientation of eye 6 and light projector 41, i.e. the centring of the light projector 41 to the eye 6, is effected by moving the patient's bed or the base station 2 in the x-direction and y-direction of a horizontal positioning plane (generally parallel to the ground surface on which the base station 2 is arranged).

In the embodiment according to Figures 2 and 3, the support arm 3 is connected movably to the base station 2 such that the support arm 3, for orientation of the light projector 41 to the eye 6, can be moved in translation in the x-direction and y-direction of a horizontal positioning plane relative to the base station 2. The translatory movement is effected by means of translatory movement drivers or manually via corresponding guides.

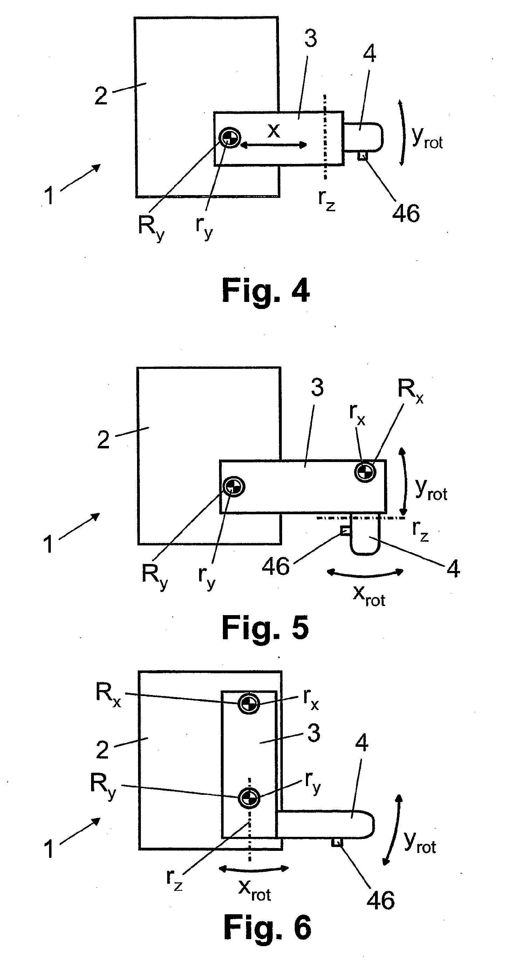

In the embodiment according to Figure 4, the support arm 3 is connected to the base station 2 via the hinge Ry, e.g. a pivot hinge or pivot joint. The hinge Ry permits a rotation yᵣₒₜ of the support arm 3 about the vertical rotation axis r_{y}. As is shown schematically in Figure 4, the hinge R_{y} is arranged and configured such that, by the rotation yᵣₒₜ of the support arm 3 about the rotation axis r_{y}, a horizontal movement is permitted in the y-direction for orienting the light projector 41 to the eye 6. The orientation of the light projector 41 in the x-direction is effected by means of translatory movement, as in Figures 2 and 3.

In the embodiment according to Figure 5, the orientation of the light projector 41 is effected by rotation both in the x-direction and also in the y-direction. In the embodiment according to Figure 5, the application head 4 is connected to that end of the support arm 3 directed away from where the support arm 3 is connected to the base station 2, in particular also via the hinge Rₓ. The hinge Rₓ permits a rotation xᵣₒₜ of the application head 4 about the vertical axis rₓ. As is shown schematically in Figure 5, the hinge Rₓ is arranged and configured such that, by the rotation xᵣₒₜ of the application head 4 about the rotation axis rₓ, a horizontal movement in the x-direction is permitted for orienting the light projector 41 to the eye 6. The orientation of the light projector 41 in the y-direction is effected by means of a rotary movement, as in Figure 4.

In the embodiment according to Figure 6, the orientation of the light projector 41 is effected by rotation both in the x-direction and also in the y-direction, as in Figure 5. In the embodiment according to Figure 6, however, the hinge R_{z} or rotation axis r_{z} is turned through 90° in relation to the arrangement according to Figure 5. The movement in the x-direction is effected by a rotation xᵣₒₜ of the support arm 3 about the vertical rotation axis rₓ of the hinge Rₓ, which connects the support arm 3 to the base station 2. The movement in the y-direction is effected by a rotation yᵣₒₜ of the application head 4 about the vertical axis ry of the hinge R_{y} which connects the application head 4 to the support arm 3.

The positioning means for the rotatory and/or translatory movement of the support arm 3 for horizontal orientation of the application head 4 and of the light projector 41 with translatory movements in the x-direction and y-direction and/or with rotary movements xᵣₒₜ and yᵣₒₜ can be configured for manual movement and/or by means of movement drivers.

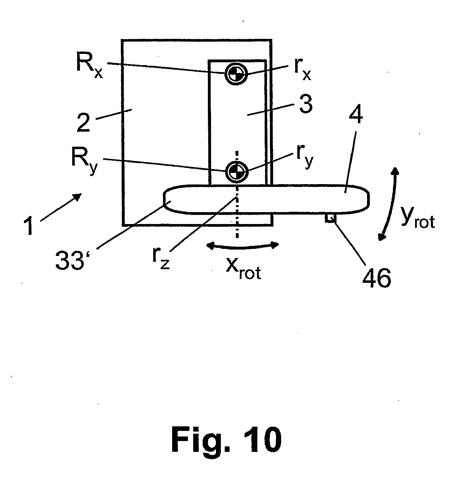

Although this is only shown schematically in Figure 2, the ophthalmological apparatus 1 comprises weight compensation means 33 which are connected to the application head 4, for example adjustable counter-weights 33', as illustrated in Figures 10 to 14, or springs. The weight compensation means 33 are preferably configured such that they only partially balance out the masses rotating about the rotation axis r_{z}, such that the application head 4 can be placed with a defined application force onto the eye 6.

Although this is only shown schematically in Figure 1, the ophthalmological apparatus 1 additionally comprises a control unit 23 arranged in the base station 2. The control unit 23 ensures that the beam-deflecting means 31 are not activated when the application head 4 is moved for placement onto the eye 6. The control unit 23 is also designed for controlling the beam deflection and for controlling and monitoring the movement drivers. The control unit 23 additionally comprises safety functions for monitoring of forces, movements and beam parameters.

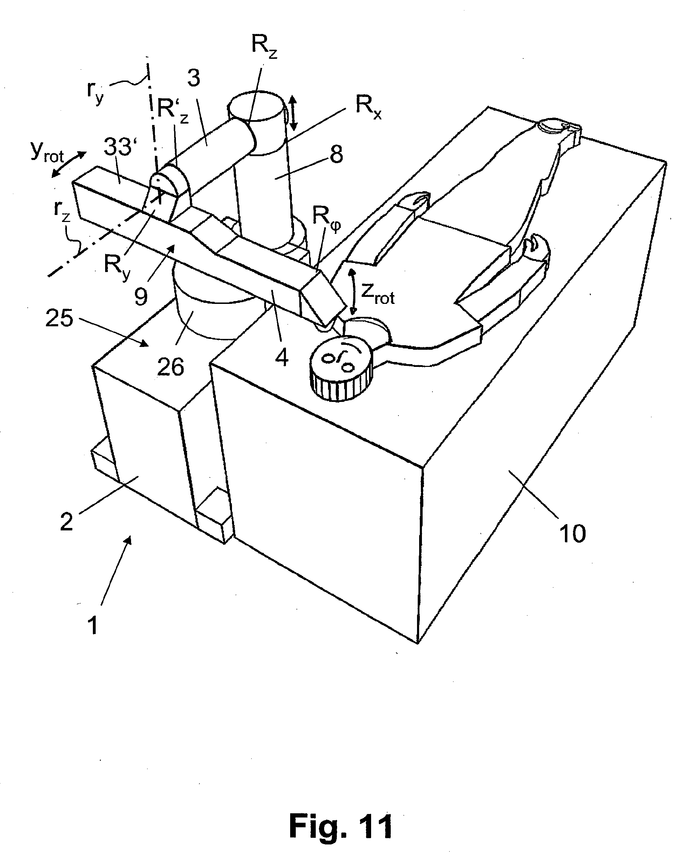

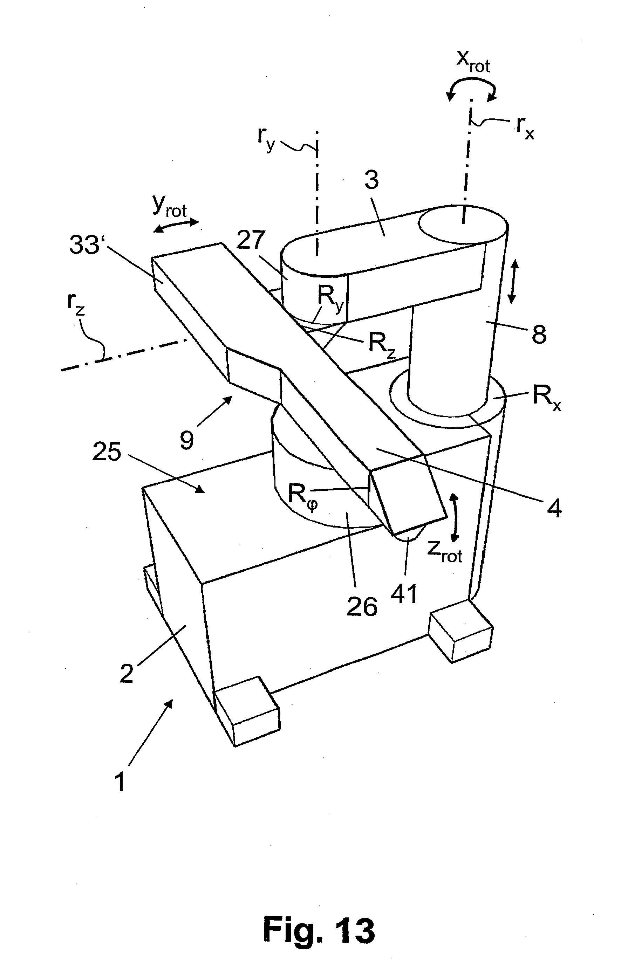

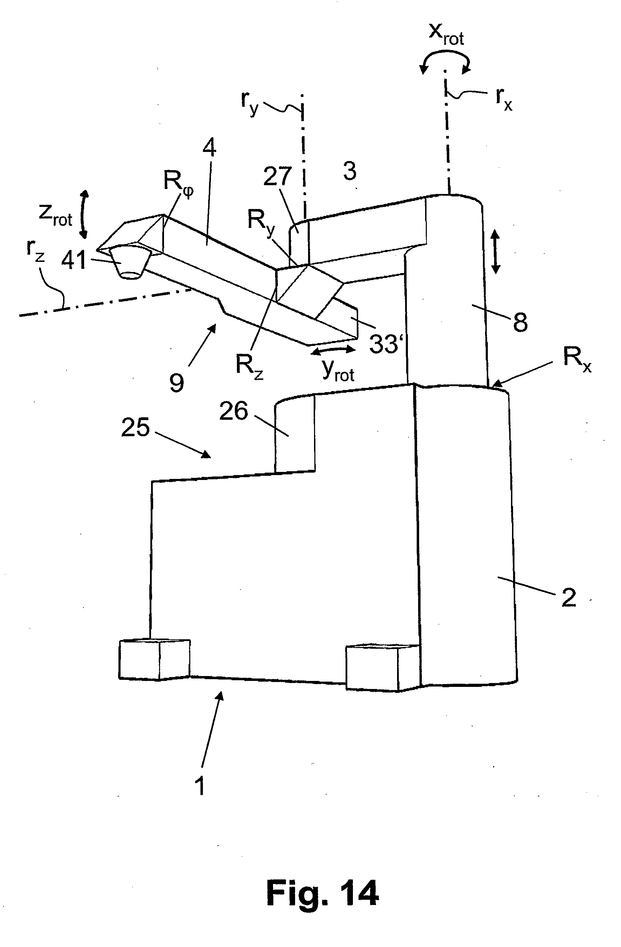

In one embodiment, the ophthalmological apparatus 1 moreover comprises height-defining means 22 for determining a vertical position of the eye 6, for example a camera or alignment laser beam(s) from a projector attached to the support arm 3, and the base station 2 comprises height-positioning means 24, e.g. translatory movement drivers, for setting a vertical basic position of the base station 2 and of the associated support arm 3. Thus, by setting the vertical position, the height-positioning means 24 make it possible to adjust the height of the horizontal positioning plane for the support arm 3. For a set vertical position, the support arm 3 is limited to defined horizontal movements through translation and/or rotation. The support arm 3 can be moved laterally at set heights but keeps an essentially horizontal orientation. The height-positioning means 24 are controlled, for example, by the control unit 23 on the basis of a vertical position of the eye 6 determined by the height-defining means 22. Manual setting of the basic position is also possible. By setting the basic position, the movement stroke required for the application head 4 can be reduced to 10-20 mm, for example. In the embodiments according to Figures 11 to 14, the support arm 3 is mounted on a column 8. The column 3 is vertically movable for setting the vertical position of the support arm 3. Preferably, at least in the embodiments of Figures 13 and 14, column 8 is connected to the base station 2 via a hinge Rₓ, particularly a pivot hinge or a pivot joint, and can be rotated about its vertical axis rₓ.

In the embodiments according to Figures 10 to 14, attached to the application head 4 are counter-weights 33' for balancing the application head 4, as described above with reference to Figure 2. Preferably, the application head 4 and the attached counter-weights 33' are formed as an elongated member 9.

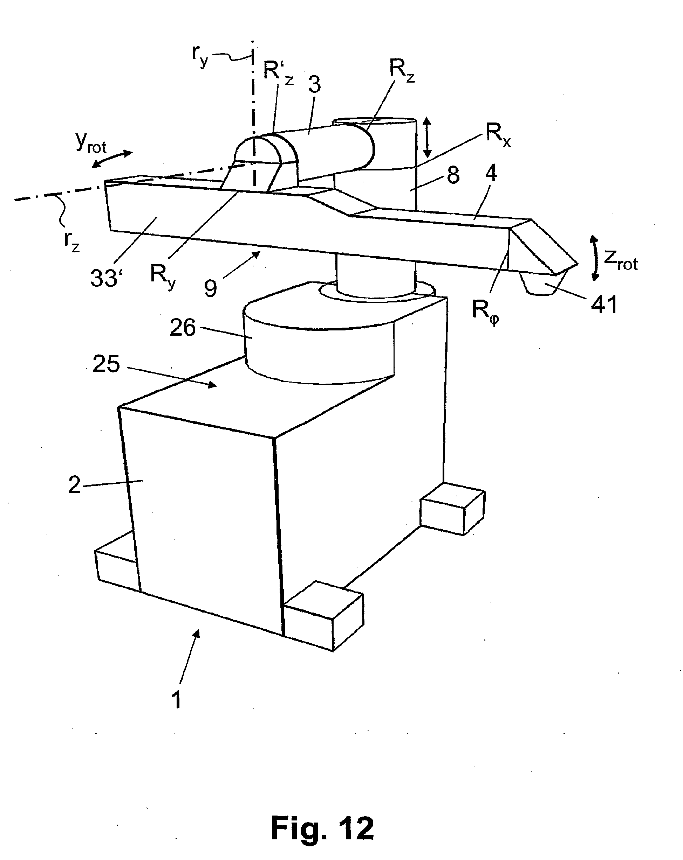

The embodiments according to Figures 10-14 correspond essentially to the embodiment of Figures 5 or 6. Figures 10-14 illustrate more clearly, however, that the elongated member 9 with the application head 4 and the attached counter-weights 33' can be rotated freely about the horizontal rotation axis r_{z} without being blocked by either base station 2, support arm 3, or column 8. As illustrated in Figures 11 to 14, the base station 2 has a recess 25 for letting the elongated member 9 pass. At least, the elongated member 9 can be rotated into a vertical, upright position, whereby the counter-weights 33' are placed close to the base station 2 in the recess 25, and the application head 4 and the light projector 41 are on top, directed away from the base station 2. In the embodiments of Figures 10-14, the base station 2 can be positioned on the left or right hand side of the patient in that, through appropriate positioning of the elongated member 9, the application head 4 can be arranged over the patient's eye, either perpendicularly to or aligned with the patient's body. For example, in the embodiments of Figures 11 and 12, the application head 4 can be positioned on either side of the patient, either perpendicularly to or aligned with the patients body, by rotating the elongated member 9 about the vertically oriented rotation axis r_{y}. Alternatively, in the embodiments of Figures 10-14, through rotation of the elongated member 9 about the horizontal rotation axis r_{z} in combination with a respective rotation of the projection part 4B about the longitudinal axis r_{ϕ}, the application head 4 can be arranged over the patient's eye either perpendicularly to or aligned with the patient's body. Preferably, the ophthalmological apparatus 1 comprises orientation detectors for determining individually or concurrently the orientation of support arm 3, elongated member 9, application head 4, and/or projection part 4B. The orientation detectors are connected to a processor which is configured to adjust and compensate settings of scanners, cameras and measurement modules based on the detected orientation.

In the embodiments of Figures 10-14 the elongated member 9 is connected to the support arm 3 via a hinge R_{y} having a rotation axis r_{y} oriented perpendicularly to the longitudinal axis of the support arm 3. The latter hinge R_{y} enables lateral movement of the elongated member 9, and thus the application head 4, through rotation yᵣₒₜ about the rotation axis r_{y}. As is illustrated in Figures 11-14, the elongated member 9, including the application head 4 and the counter-weights 33', is arranged offset from the support arm 3 so that the elongated member 9 can be rotated freely about the rotation axis r_{y} without being blocked by the support arm 3. To allow for free rotation of the elongated member 9 about the rotation axis r_{y}, while the elongated member 9 is in a vertical, upright position, the recess 25 is formed/bordered correspondingly with a rounded face 26.

In the embodiment according to Figures 11 and 12, either the support arm 3 is connected movably to the base station 2, e.g. to column 8, via the horizontally oriented hinge R_{z}, e.g. a pivot hinge or a pivot joint, or, preferably, the elongated member 9 is connected to the support arm 3 via the horizontally oriented hinge R_{z}' on the end of the support arm 3, directed away from where the support arm 3 is connected fixedly to the base station 2, e.g. to column 8. Essentially, the horizontally oriented rotation axis r_{z} is aligned with the longitudinal axis of the support arm 3 and enables a rotation of the elongated member 9 about the longitudinal axis of the support arm 3. While the rotatable support arm 3 and/or a rotation of the elongated member 9 enable a rotation zᵣₒₜ of the application head 4 about the horizontal axis r_{z}, hinge R_{y} enables lateral movement of the application head 4 parallel to the longitudinal axis of the support arm 3.

In the embodiment according to Figures 13 and 14, the support arm 3 is connected fixedly to the base station 2, e.g. to column 8. On the other end of the support arm 3, directed away from where the support arm 3 is connected to the base station 2, the elongated member 9 is connected to the support arm 3 via the horizontally oriented hinge R_{z}. The horizontally oriented hinge R_{z} is connected to the support arm 3 via the vertically oriented hinge R_{y} having a vertical rotation axis r_{y} arranged perpendicularly to the longitudinal axis of the support arm 3. While the horizontally oriented hinge R_{z} enables a rotation zᵣₒₜ of the application head 4 about the horizontal axis r_{z}, hinge R_{y} enables lateral movement of the application head 4 parallel to the longitudinal axis of the support arm 3. To allow for free rotation of the elongated member 9 about the rotation axis r_{y}, while the elongated is in a non-horizontal position, the support arm 3 is provided with a rounded face 27.

Preferably, the ophthalmological apparatus 1 comprises further locks for fixing or releasing a position of rotation of rotatable members about their respective rotation axis, specifically there are defined positions for transporting or parking the ophthalmological apparatus 1 and for equipping the ophthalmological apparatus 1 with different optical and/or mechanical components. Moreover, for manual handling and control, the ophthalmological apparatus 1 comprises handles and/or gripping structures provided on the movable parts, particularly on the support arm 3, the elongated member 9, the application head 4, and/or the projection part 4B.

It shall be stated here that the term "hinge" is not intended to limit the scope of the invention, but to be as broad as the German term "Drehgelenk", i.e. a pivot joint connecting two mechanical bodies and enabling rotation of at least one of these bodies, relative to the other body, about a defined rotation axis.

## Claims

1. Ophthalmological apparatus (1), comprising:
a base station (2) with a light source (21) for generating light pulses,
a support arm (3) mounted on the base station (2),
an application head (4) mounted on the support arm (3) and placeable onto an eye (6), the application head including a light projector (41) adapted for focused projection of the light pulses for punctiform breakdown of eye tissue,
an optical transmission system (22) adapted to transmit the light pulses from the base station (2) through the support arm (3) to the application head (4), and
beam-deflecting means (31) arranged in the application head (4) and configured to deflect the light pulses in at least two scanning directions.
**characterized in that**
the support arm (3) is of rigid design with a constant horizontal orientation and, at one end, has a hinge (R_{z}) with an essentially horizontally oriented rotation axis (r_{z}), the hinge (R_{z}) being mounted in such a way that the application head (4) is adapted to be rotated with a rotation (zᵣₒₜ) extending about the horizontal rotation axis (r_{z}) for lowering and placing the application head (4) onto the eye (6).

2. Apparatus (1) according to Claim 1, **characterized in that** the hinge (R_{z}') is arranged on that end of the support arm (3) directed away from where the support arm (3) is mounted on the base station (2), and **in that** the application head (4) is connected movably to the support arm (3) via the hinge (R_{z}').

3. Apparatus (1) according to one of Claims 1 or 2, **characterized in that** the apparatus (1) comprises positioning means for rotary and/or translatory movement of the support arm (3) parallel to a positioning plane for the positioning of the application head (4) over the eye (6).

4. Apparatus (1) according to one of Claims 1 to 3, **characterized in that** the hinge (R_{z}) is connected to the support arm (3) via a further hinge (R_{y}), the further hinge (R_{y}) having a vertically oriented rotation axis (r_{y}) and enabling a rotation (yᵣₒₜ) of the application head (4) about this vertical rotation axis (r_{y}).

5. Apparatus according to Claim 1, **characterized in that** the support arm (3) is connected movably to the base station (2) via the hinge (R_{z}), and **in that** the horizontally oriented rotation axis (r_{z}) is aligned with the longitudinal axis of the support arm (3) and enables a rotation of the support arm (3) about its longitudinal axis.

6. Apparatus (1) according to one of Claims 2 or 5, **characterized in that** the application head (4) is connected to the support arm (3) via a further hinge (R_{y}), the further hinge having a rotation axis (r_{y}) oriented perpendicularly to the longitudinal axis of the support arm (3) and enabling a rotation of the application head (4) about this rotation axis (r_{y}).

7. Apparatus (1) according to one of Claims 1 to 6, **characterized in that** the support arm (3) is connected to the base station (2) via a pivot joint having a vertically oriented rotation axis (rₓ, r_{y}) and enabling a rotation of the support arm (3) about this vertical rotation axis (rₓ, r_{y}).

8. Apparatus (1) according to one of Claims 1 to 7, **characterized in that** the light projector (41) comprises a first lens system (43) in a projection part (4B) of the application head (4) oriented in the projection direction (v), and **in that** the light projector (41) comprises a second lens system (42) in a feed part (4A) of the application head (4) angled away from the projection part (4b) and directed towards the support arm (3), said first and second lens systems being coupled via a deflecting mirror (45).

9. Apparatus (1) according to one of Claims 1 to 8, **characterized in that** the apparatus (1) comprises height-defining means (22) for determining a vertical position of the eye (6), and **in that** the base station (2) comprises height-positioning means (24) for setting a vertical basic position of the support arm (3).

10. Apparatus (1) according to one of Claims 1 to 9, **characterized in that** the application head (4) has at least one of grips and grip structures (46), **in that** the application head (4) comprises a contact body which can be placed onto the eye (6), is transparent to light at least in parts and is configured and arranged such that it sets a contacted area of the eye (6) equidistant to a work surface, **in that** the application head (4) has securing means for fixing the application head (4) on the eye (6) by underpressure, and **in that** the light source (21) comprises a femtosecond laser.

11. Apparatus (1) according to one of Claims 1 to 12, **characterized in that** the base station (2) comprises a column (8), **in that** the support arm (3) is mounted on the column (8), and **in that** the column (8) is vertically movable for setting a vertical position of the support arm (3).

12. Apparatus (1) according to one of Claims 1 to 11, **characterized in that** the application head (4) is formed as an elongated member (9), **in that** counter-weights (33') are integrated in the elongated member (9) to at least partially balance out the application head (4) above the horizontally oriented rotation axis (r_{z}), and **in that** the elongated member (9) is connected to the support arm (3) via a hinge (R_{y}) having a rotation axis (r_{y}) oriented perpendicularly to the longitudinal axis of the support arm (3).

13. Apparatus (1) according to claim 12, **characterized in that** the hinge (R_{z}) with the horizontally oriented rotation axis (r_{z}) is mounted in such a way that the application head (4) can be rotated into a vertical position.

## Patentansprüche

1. Ophthalmologische Vorrichtung (1), die Folgendes umfasst:
eine Basisstation (2) mit einer Lichtquelle (21) zur Erzeugung von Lichtpulsen,
einen Trägerarm (3), der an der Basisstation angebracht ist (2),
einen Anlegekopf (4), der an dem Trägerarm (3) angebracht ist und auf ein Auge (6) setzbar ist, wobei der Anlegekopf einen Lichtprojektor (41) enthält, der zur fokussierten Projektion der Lichtpulse für punktförmigen Abbau von Augengewebe ausgelegt ist,
ein optisches Übertragungssystem (22), das dazu ausgelegt ist, die Lichtpulse von der Basisstation (2) durch den Trägerarm (3) an den Anlegekopf (4) zu übertragen, und
Strahlenablenkungsmittel (31), die in dem Anlegekopf (4) angeordnet und dazu konfiguriert sind, die Lichtpulse in mindestens zwei Scanrichtungen abzulenken,
**dadurch gekennzeichnet, dass**
der Trägerarm (3) einen steifen Aufbau mit einer konstanten horizontalen Orientierung hat und an einem Ende ein Drehgelenk (R_{z}) mit einer im Wesentlichen horizontal orientierten Drehachse (r_{z}) aufweist, wobei das Drehgelenk (R_{z}) derart angebracht ist, dass der Anlegekopf (4) dazu ausgelegt ist, mit einer Drehung (zᵣₒₜ), die sich um die horizontale Drehachse (r_{z}) erstreckt, gedreht werden zu können, um den Anlegekopf (4) auf das Auge (6) zu senken und abzulegen.

2. Vorrichtung (1) nach Anspruch 1, **dadurch gekennzeichnet, dass** das Drehgelenk (R_{z}') an dem Ende des Trägerarms (3) angeordnet ist, das von der Stelle, an der der Trägerarm (3) an der Basisstation (2) angebracht ist, weggerichtet ist, und dass der Anlegekopf (4) über das Drehgelenk (R_{z}') bewegbar mit dem Trägerarm (3) verbunden ist.

3. Vorrichtung (1) nach einem der Ansprüche 1 oder 2, **dadurch gekennzeichnet, dass** die Vorrichtung (1) Positioniermittel für eine Dreh- und/oder Translationsbewegung des Trägerarms (3) parallel zu einer Positionsebene für die Positionierung des Anlegekopfs (4) über das Auge (6) umfasst.

4. Vorrichtung (1) nach einem der Ansprüche 1 bis 3, **dadurch gekennzeichnet, dass** das Drehgelenk (R_{z}) über ein weiteres Drehgelenk (R_{y}) mit dem Trägerarm (3) verbunden ist, wobei das weitere Drehgelenk (R_{y}) eine vertikal orientierte Drehachse (r_{y}) aufweist und eine Drehung (yᵣₒₜ) des Anlegekopfs (4) um diese vertikale Drehachse (r_{y}) ermöglicht.

5. Vorrichtung nach Anspruch 1, **dadurch gekennzeichnet, dass** der Trägerarm (3) über das Drehgelenk (R_{z}) bewegbar mit der Basisstation (2) verbunden ist, und dass die horizontal orientierte Drehachse (r_{z}) mit der Längsachse des Trägerarms (3) ausgerichtet ist und eine Drehung des Trägerarms (3) um seine Längsachse ermöglicht.

6. Vorrichtung (1) nach einem der Ansprüche 2 oder 5, **dadurch gekennzeichnet, dass** der Anlegekopf (4) über ein weiteres Drehgelenk (R_{y}) mit dem Trägerarm (3) verbunden ist, wobei das weitere Drehgelenk eine Drehachse (r_{y}) aufweist, die senkrecht zur Längsachse des Trägerarms (3) orientiert ist und eine Drehung des Anlegekopfes (4) um diese Drehachse (r_{y}) ermöglicht.

7. Vorrichtung (1) nach einem der Ansprüche 1 bis 6, **gekennzeichnet dadurch, dass** der Trägerarm (3) über ein Drehgelenk, das eine vertikal orientierte Drehachse (rₓ, r_{y}) aufweist und eine Drehung des Trägerarms (3) um diese vertikale Drehachse (rₓ, r_{y}) ermöglicht, mit der Basisstation (2) verbunden ist.

8. Vorrichtung (1) nach einem der Ansprüche 1 bis 7, **gekennzeichnet dadurch, dass** der Lichtprojektor (41) in einem Projektionsteil (4B) des Anlegekopfes (4), das in die Projektionsrichtung (v) orientiert ist, ein erstes Linsensystem (43) umfasst, und dass der Lichtprojektor (41) in einem Zuführteil (4A) des Anlegekopfes (4), das von dem Projektionsteil (4B) abgewinkelt und zum Trägerarm (3) hin gerichtet ist, ein zweites Linsensystem (42) umfasst, wobei das erste und zweite Linsensystem über einen Ablenkungsspiegel (45) gekoppelt sind.

9. Vorrichtung (1) nach einem der Ansprüche 1 bis 8, **gekennzeichnet dadurch, dass** die Vorrichtung (1) Höhendefinitionsmittel (22) zur Bestimmung einer vertikalen Position des Auges (6) umfasst, und dass die Basisstation (2) Höhenpositionierungsmittel (24) zur Einstellung einer vertikalen Grundposition des Trägerarms (3) umfasst.

10. Vorrichtung (1) nach einem der Ansprüche 1 bis 9, **gekennzeichnet dadurch, dass** der Anlegekopf (4) Griffe und/oder Griffstrukturen (46) aufweist, dass der Anlegekopf (4) einen Kontaktkörper umfasst, der auf das Auge (6) gesetzt werden kann, zumindest teilweise gegenüber Licht transparent und derart konfiguriert und angeordnet ist, dass er einen Bereich des Auges (6), mit dem er in Kontakt getreten ist, äquidistant zu einer Arbeitsfläche einstellt, dass der Anlegekopf (4) Sicherungsmittel zur Befestigung des Anlegekopfes (4) auf dem Auge (6) durch Unterdruck aufweist, und dass die Lichtquelle (21) einen Femtosekunden-Laser umfasst.

11. Vorrichtung (1) nach einem der Ansprüche 1 bis 12, **gekennzeichnet dadurch, dass** die Basisstation (2) eine Säule (8) umfasst, dass der Trägerarm (3) an der Säule (8) angebracht ist, und dass die Säule (8) zur Einstellung einer vertikalen Position des Trägerarms (3) vertikal bewegbar ist.

12. Vorrichtung (1) nach einem der Ansprüche 1 bis 11, **gekennzeichnet dadurch, dass** der Anlegekopf (4) als längliches Glied (9) ausgebildet ist, dass Gegengewichte (33') in dem länglichen Glied (9) integriert sind, um den Anlegekopf (4) über der horizontal orientierten Drehachse (r_{z}) zumindest teilweise auszubalancieren, und dass das längliche Glied (9) über ein Drehgelenk (R_{y}) mit einer Drehachse (r_{y}), die senkrecht zur Längsachse des Trägerarms (3) orientiert ist, mit dem Trägerarm (3) verbunden ist.

13. Vorrichtung (1) nach Anspruch 12, **gekennzeichnet dadurch, dass** das Drehgelenk (R_{z}) mit der horizontal orientierten Drehachse (r_{z}) derart angebracht ist, dass der Anlegekopf (4) in eine vertikale Position gedreht werden kann.

## Revendications

1. Appareil ophtalmologique (1), comprenant :
une station de base (2) avec une source de lumière (21) pour générer des impulsions de lumière,
un bras support (3) monté sur la station de base (2),
une tête d'application (4) montée sur le bras support (3) pouvant être placée sur un oeil (6), la tête d'application comprenant un projecteur de lumière (41) conçu pour une projection concentrée des impulsions de lumière pour une dégradation punctiforme du tissu de l'oeil,
un système de transmission optique (22) conçu pour transmettre les impulsions de lumière depuis la station de base (2) par l'intermédiaire du bras support (3) à la tête d'application (4), et
un moyen de déviation de faisceau (31) disposé dans la tête d'application (4) et configuré pour dévier les impulsions de lumière dans au moins deux directions de balayage,
**caractérisé en ce que** :
le bras support (3) est de conception rigide avec une orientation horizontale constante et, à une extrémité, dispose d'une charnière (R_{z}) avec un axe de rotation (r_{z}) orienté essentiellement horizontalement, la charnière (R_{z}) étant montée de telle manière que la tête d'application (4) soit conçue pour tourner avec une rotation (zᵣₒₜ) s'étendant autour de l'axe de rotation (r_{z}) horizontal pour baisser et placer la tête d'application (4) sur l'oeil (6).

2. Appareil (1) selon la revendication 1, **caractérisé en ce que** la charnière (R_{z}') est disposée sur l'extrémité du bras support (3) dirigée dans l'autre direction que l'emplacement où le bras support (3) est monté sur la station de base (2), et **en ce que** la tête d'application (4) est connectée, de manière mobile, au bras support (3) par l'intermédiaire de la charnière (R_{z}').

3. Appareil (1) selon l'une des revendications 1 ou 2, **caractérisé en ce que** l'appareil (1) comprend un moyen de positionnement pour des mouvements de rotation et/ou de translation du bras support (3) parallèles à un plan de positionnement pour le positionnement de la tête d'application (4) sur l'oeil (6).

4. Appareil (1) selon l'une des revendications 1 à 3, **caractérisé en ce que** la charnière (R_{z}) est connectée au bras support (3) par l'intermédiaire d'une charnière supplémentaire (R_{y}), la charnière supplémentaire (R_{y}) ayant un axe de rotation (r_{y}) orienté verticalement et permettant une rotation (yᵣₒₜ) de la tête d'application (4) autour de cet axe de rotation vertical (r_{y}).

5. Appareil (1) selon la revendication 1, **caractérisé en ce que** le bras support (3) est connecté, de manière mobile, à la station de base (2) par l'intermédiaire de la charnière (R_{z}), et **en ce que** l'axe de rotation (r_{z}) orienté horizontalement est aligné avec l'axe longitudinal du bras support (3) et permet une rotation du bras support (3) autour de son axe longitudinal.

6. Appareil (1) selon l'une des revendications 2 ou 5, **caractérisé en ce que** la tête d'application (4) est connectée au bras support (3) par l'intermédiaire d'une charnière supplémentaire (R_{y}), la charnière supplémentaire ayant un axe de rotation (r_{y}) orienté perpendiculairement à l'axe longitudinal du bras support (3) et permettant une rotation de la tête d'application (4) autour de cet axe de rotation (r_{y}).

7. Appareil (1) selon l'une des revendications 1 à 6, **caractérisé en ce que** le bras support (3) est connecté à la station de base (2) par l'intermédiaire d'une articulation à pivot ayant un axe de rotation (rₓ, r_{y}) orienté verticalement et permettant une rotation du bras support (3) autour de cet axe de rotation (rₓ, r_{y}) vertical.

8. Appareil (1) selon l'une des revendications 1 à 7, **caractérisé en ce que** le projecteur de lumière (41) comprend un premier système de lentille (43) dans une partie de projection (4B) de la tête d'application (4) orientée dans la direction de projection (v), et **en ce que** le projecteur de lumière (41) comprend un second système de lentille (42) dans une partie d'alimentation (4A) de la tête d'application (4) s'écartant d'un certain angle de la partie de projection (4B) et dirigé vers le bras support (3), lesdits premier et second systèmes de lentille étant couplés par l'intermédiaire d'un miroir déflecteur (45).

9. Appareil (1) selon l'une des revendications 1 à 8, **caractérisé en ce que** l'appareil (1) comprend un moyen de définition de hauteur (22) pour déterminer une position verticale de l'oeil (6), et **en ce que** la station de base (2) comprend un moyen de positionnement de hauteur (24) pour définir une position de base verticale du bras support (3).

10. Appareil (1) selon l'une des revendications 1 à 9, **caractérisé en ce que** la tête d'application (4) dispose au moins d'une prise parmi des poignées ou des structures de poignées (46), **en ce que** la tête d'application (4) comprend un corps de contact qui peut être placé sur l'oeil (6), est transparent à la lumière, au moins au niveau de certaines parties, et est configuré et disposé de manière à ce qu'il définisse une zone en contact de l'oeil (6) équidistante d'une surface de travail, **en ce que** la tête d'application (4) dispose d'un moyen de sécurité pour fixer la tête d'application (4) sur l'oeil (6) par dépression, et **en ce que** la source de lumière (21) comprend un laser femtoseconde.

11. Appareil (1) selon l'une des revendications 1 à 10, **caractérisé en ce que** la station de base (2) comprend une colonne (8), **en ce que** le bras support (3) est monté sur la colonne (8), et **en ce que** la colonne (8) est déplaçable verticalement pour définir une position verticale du bras support (3).

12. Appareil (1) selon l'une des revendications 1 à 11, **caractérisé en ce que** la tête d'application (4) se présente sous la forme d'un élément allongé (9), **en ce que** des contrepoids (33') sont intégrés dans l'élément allongé (9) pour, au moins partiellement, équilibrer la tête d'application (4) au-dessus de l'axe de rotation (r_{z}) orienté horizontalement, et **en ce que** l'élément allongé (9) est connecté au bras support (3) par l'intermédiaire d'une charnière (R_{y}) ayant un axe de rotation (r_{y}) orienté perpendiculairement à l'axe longitudinal du bras support (3).

13. Appareil (1) selon la revendication 12, **caractérisé en ce que** la charnière (R_{z}) ayant l'axe de rotation (r_{z}) orienté horizontalement est montée de telle manière que la tête d'application (4) puisse être tournée dans une position verticale.
